# EUROPEAN PATENT SPECIFICATION

(11) **EP 3 129 073 B1**
(45) Date of publication and mention of the grant of the patent: **31.10.2018**
(21) Application number: 15708223.1
(22) Date of filing: 06.03.2015
(51) Int. Cl.: A61L 27/18, A61L 27/50

(54) **BIODEGRADABLE POLYESTERAMIDE USED FOR THE TREATMENT OF ARTHRITIC DISORDERS**
BIOLOGISCH ABBAUBARES POLYESTERAMID ZUR BEHANDLUNG VON ARTHRITISCHEN ERKRANKUNGEN
POLYESTERAMIDES BIODÉGRADABLES UTILISÉS POUR LE TRAITEMENT DE TROUBLES ARTHRITIQUES

(30) Priority: 08.04.2014 EP 14163862
(43) Date of publication of application: 15.02.2017
(73) Proprietor: DSM IP Assets B.V., 6411 TE Heerlen (NL)
(72) Inventor: THIES, Jens Christoph, 6100 AA Echt (NL); SCHUMANN, Detlef Olaf Alexander, 6100 AA Echt (NL); MIHOV, George, 6100 AA Echt (NL); BERARD, Julien François, 6100 AA Echt (NL)
(74) Representative: DSM Intellectual Property
(86) International application number: PCT/EP2015/054785
(87) International publication number: WO 2015/154924

(56) References cited:
- US-A1- 2008 299 174
- US-A1- 2012 165 760

## Description

The present invention relates to a formulation injectable in a human or veterinary patient comprising microparticles sized for injection, wherein the microparticles comprise an analgesic or a disease-modifying antirheumatic drug, and one or more biocompatible polymers comprising a biodegradable polyesteramide co-polymer comprising structural Formula (I) for use in the treatment of arthritic disorders. The present invention further relates to the formulation for use in the treatment of arthritic disorders being administered by 1 or 2 injections per year.

Arthritic disorders are related with a strong inflammatory reaction causing pain and discomfort for a patient. Pain is associated with many medical conditions and affects millions of people. The American Pain Foundation reports that over 50 million Americans suffer from chronic pain including 20 percent of individuals aged 60 and over who are affected by arthritis or back pain. Furthermore, nearly 25 million Americans experience acute pain due to injuries or surgical procedures each year. The cost involved in the management of pain has been estimated at $100 billion each year. In addition to its economic burden, pain has a tremendous effect on the quality of life of affected individuals and is one of the most common causes of acute and chronic disabilities.

The human body perceives pain when body tissues, including nerve fibers, are damaged by pathogens, trauma, inflammatory conditions or noxious stimuli ranging from harmful or noxious mechanical stimuli, hot and/or cold stimuli, or chemical stimuli. Mast cells associated with damaged tissue and nerve fibers initiate the inflammation process by secreting inflammatory mediators, e.g. Tumor Necrosis Factor-alpha (TNF-alpha), histamine, Interleukin-1 (IL-I), IL-6, IL-8, and nerve growth factors (NGF). These mediators cause other cells, such as monocytes, neutrophiles, and similar cells, to migrate to the trauma site. Further, these mediators also help some of the white cells, such as phagocytes, to activate their own inflammatory mediators. Inflammatory mediators, such as, NGFs secreted by damaged or irritated nerve cells and fibers have been shown to increase the number of active nerve fibers.

Inflammatory mediators involved in pain are allied with various disorders that may include without limitation: arthritis for example osteoarthritis, rheumatoid arthritis, low back pain, psoriatic arthritis, autoimmune arthritis, septic arthritis or synovitis. In general, inflammation is a normal and essential response to any noxious stimulus and may vary from a localized to a generalized response. The inflammatory response generally follows a sequence of events that include, 1) an initial injury causing release of inflammatory mediators, such as, histamine, serotonin, leukokinins, SRS-A, lysosomal enzymes, lymphokinins, prostaglandins, etc.; 2) vasodilation, including increased vascular permeability and exudation; 3)leukocyte migration, chemotaxis, and phagocytosis; and 4) proliferation of connective tissue cells.

Arthritis is a form of arthritic disorder that involves inflammation of one or more joints. There are over 100 different forms of arthritis. The most common form, osteoarthritis (degenerative joint disease) is a result of trauma to the joint, infection of the joint or age. Other arthritis forms are rheumatoid arthritis, psoriatic arthritis and related autoimmune diseases. Septic arthritis is caused by joint infection.

Osteoarthritis can affect both the larger and the smaller joints of the body including the hands, wrists, shoulder, feet, back, hip, spine or knee. Osteoarthritis causes cartilage to degenerate and eventually causes the two opposing bones to erode into each other. Initially, the condition starts with minor pain during activities, but soon the pain can be continuous and even occur while in a state of rest. Osteoarthritis typically affects the weight-bearing body parts, such as the hips, knees, spine, as well as pelvis and shoulders. Unlike rheumatoid arthritis, osteoarthritis is most commonly a disease of the elderly. More than 30 percent of women have some degree of osteoarthritis by age 65. Risk factors for osteoarthritis include prior joint trauma, obesity, and a sedentary lifestyle.

Osteoarthritis, like rheumatoid arthritis, cannot be cured so far. Pain medications are widely required by individuals with osteoarthritis, especially anti-inflammatory drugs are known in the art as being useful for treating inflammation. Within the anti-inflammatory drugs corticosteroids are often used to treat pain. Corticosteroids influence all tissues of the body and produce various cellular effects. These steroids regulate carbohydrate, lipid, protein biosynthesis and metabolism, and water and electrolyte balance. Corticosteroids influencing cellular biosynthesis or metabolism are referred to as glucocorticoids while those affecting water and electrolyte balance are mineralocorticoids.

Corticosteroids injections are often used to relief pain in for example osteoarthritis. The clinical benefit of these injections is however controversy because complications have been associated with large bolus steroid injections. Moreover these products are short acting and only provide a short term relief of pain.

US2012282298 discloses a long-term controlled or sustained release formulation of a Class B corticosteroid comprising a lactic acid-glycolic acid copolymer micro-particles used for the treatment of osteoarthritis, rheumatoid arthritis, acute gouty arthritis or synovitis. Disadvantage of this formulation is however that lactic acid-glycolic acid copolymers degrade hydrolytically whereby acidic byproducts are released during the polymer degradation.

It is the object of the present invention to overcome the above mentioned drawbacks and to provide a local therapeutic concentration of drugs in a sustained release manner. Degenerative joint diseases often involve inflammatory flare events (induced by intense activity for example) during which the pain felt by the patient is more intense. It would be desirable to provide a release of drugs according to the intensity of the pain.

Moreover it is an object of the present invention to provide for a more efficient pain management which means an more adequate pain relief on the longer term in providing a sustained release of drugs at the therapeutic dosage.

It is a further object of the present invention to offer more stability to the drugs compared to formulations comprising polyesters such as PLGA for the release of drugs as polyesters degrade by hydrolysis and release acidic entities.

The object of the present invention is achieved in providing a formulation injectable in a human or veterinary patient for the treatment of an arthritic disorder comprising microparticles sized for injection, wherein the microparticles comprise an analgesic or a disiase-modifying antirheumatic drug and one or more biocompatible polymers comprising a biodegradable polyesteramide co-polymer comprising structural formula (I).

Surprisingly it has been found that the formulation for treatment of arthritic disorders provides a sustained release of drugs that responds to the level of inflammation and provides an adequate pain relief on the longer term which herein means multiple months. It has been found that the release of drugs from the article is dependent on the level of inflammation. This means that the release of drugs can be steered by the inflammation which is called an inflammation induced release. Furthermore, when the inflammation process slows down, then the drug release will slow down as well minimizing the unnecessary exposure of drug.

The present invention provides for using sustained- release articles comprising biodegradable polyesteramide co-polymers for local delivery of analgesics or disease-modifying antirheumatic drugs (DMARDs) to treat pain caused by inflammatory disease such arthritic disorders. More specifically the invention provides for a locally delivered dose of an analgesic or disease-modifying antirheumatic drug that can be released in a sustained way via the articles comprising the biodegradable polyesteramide copolymer near the site of a patient's source of pain.

Preferably the formulation is used for the treatment of arthritis, more preferably the formulation is used for the treatment of osteoarthritis. Most preferably the formulation is used for the treatment of osteoarthritis of the knee.

Biodegradable polyesteramide co-polymer comprising at least a diol of bicyclic-1,4:3,6-dianhydrohexitol are known from US8445007. US8445007 discloses PEA copolymers comprising two (bis-a-amino acid)-based building blocks with significant improvement in mechanical properties. Incorporation of a bicyclic-fragment of 1,4:3,6-dianhydrohexitol as the diol residue in at least one of the two bis(a-amino acid)-based building blocks in the PEA's confers high glass transition temperature (Tg) on the polymer. These PEA's are suitable for certain applications requiring a combination of hydrophobicity, relatively high glass transition temperature (Tg), and properties of variable elongation or flexibility. It is further disclosed that these PEA's can be used for the manufacturing of surgical devices such as surgical sutures, surgical screws, implantable plates, implantable rods, vascular stents or dialysis shunts. Particles are disclosed as a possible drug delivery form. This patent is however silent about a formulation used for the treatment of arthritic disorders.

The polymers represented by Formula (I) have higher glass transition temperature which is beneficial for the articles shape stability and formulation injectability. Furthermore, PEAs of such composition show a better balance between drug elution and biodegradation properties.

The polyesteramide co-polymer is a random co-polymer which comprises the following structural formula (I) 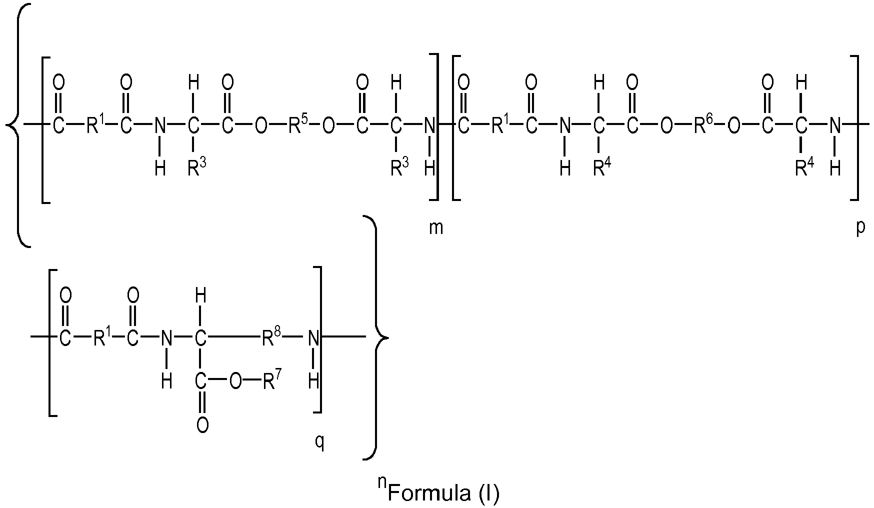 wherein
- m varies from 0.01- 0.99; p varies from 0.99-0.01; and q varies from 0.99-0.01;
- n varies from 5-100
- R₁ is independently selected from the group consisting of (C₂-C₂₀)alkylene and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl (C₁-C₆)alkyl, -(CH₂)SH, -(CH₂)₂S(CH₃), -CH₂OH,-CH(OH)CH₃, -(CH₂)₄NH₃+, -CH₂COOH, -CH₂-CO-NH₂, -CH₂CH₂-CO-NH₂,-CH₂CH₂COOH, CH₃-CH₂-CH(CH₃)-, (CH₃)₂-CH-CH₂-, H₂N-(CH₂)₄-, phenyl-CH₂-,-CH=CH-CH₃, HO-p-phenyl-CH₂-, (CH₃)₂-CH-, phenyl-NH-, NH₂-(CH₂)₃-CH₂- or NH₂-CH=N-CH=C-CH₂-
- R₅ is selected from the group consisting of (C₂-C₂₀) alkylene, (C₂-C₂₀) alkenylene.
   - R₆ is selected from bicyclic-fragments of 1,4:3,6-dianhydrohexitols of structural formula (II); 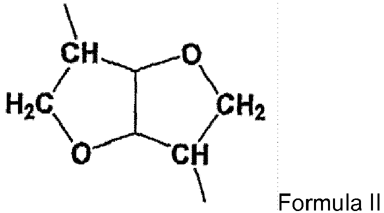
- R₇ is hydrogen, (C₆-C₁₀) aryl, (C₁-C₆) alkyl or a protecting group such as benzyl;
- R₈ is independently (C₁-C₂₀) alkylene

As used herein, the term "alkylene", refers to a straight or branched chain hydrocarbon group including methyl, ethyl, n-propyl, isopropyl, n-butyl, isobutyl, tert-butyl, n-hexyl, and the like.

The term "aryl" is used with reference to structural formulas herein to denote a phenyl radical or an ortho-fused bicyclic carbocyclic radical having about nine to ten ring atoms in which at least one ring is aromatic. Examples of aryl include, but are not limited to, phenyl, naphthyl, and nitrophenyl.

At least one of the alpha -amino acids used in the co-polymers is a natural alpha -amino acid. For example, when the R₃s or R₄s are CH₂Ph, the natural alpha-amino acid used in synthesis is L-phenylalanine. In alternatives wherein the R₃s or R₄s are CH₂-CH(CH₃)₂, the co-polymer contains the natural amino acid, leucine. By independently varying the R₃s and R₄s within variations of the two co-monomers as described herein, other natural alpha -amino acids can also be used, e.g., glycine (when the R₃s or R₄s are -H), alanine (when the R₃s or R₄s are CH₃), valine (when the R₃s or R₄s are CH(CH₃)₂), isoleucine (when the R₃s or R₄s are CH(CH₃)--CH₂--CH₃), phenylalanine (when the R₃s or R₄s are CH₂--C₆H₅), lysine (when the R₃s or R₄s (CH₂)₄-NH₂); or methionine (when the R₃s or R₄s are -(CH₂)₂S(CH₃), and mixtures thereof.

Most preferably the polyesteramide co-polymer of Formula (I) comprises m+p+q=1, q=0.25, p=0.45 whereby R₁ is -(CH₂)₈-; R₃ and R₄ in the backbone units m and p is (CH₃)₂-CH-CH₂-, R₅ is -(CH₂)₆-, R₆ is a bicyclic-fragments of 1,4:3,6-dianhydrohexitols of structural formula (II); R₇ is a benzyl group (Bz) and R₈ is -(CH₂)₄-. This polyesteramide is further referred to as PEA-III-Bz of Formula (III).

The PEA co-polymers preferably have an average number molecular weight (Mn) ranging from 15,000 to 200,000 Daltons. The PEA co-polymers described herein can be fabricated in a variety of molecular weights and a variety of relative proportions of the two bis-(alpha amino acid)-containing units and optional Lysine-based monomer of the co-polymer. The appropriate molecular weight for a particular use is readily determined by one of skill in the art. A suitable Mn will be in the order of about 15,000 to about 100,000 Daltons, for example from about 30,000 to about 80,000 or from about 35,000 to about 75,000. Mn is measured via GPC in THF with polystyrene as standard.

The basic polymerization process of polyesteramides is based on the process described by G. Tsitlanadze, et al. J. Biomater. Sci. Polym. Edn. (2004) 15:1-24, however different building blocks and activating groups were used.

The polyesteramides of Formula (I) are for example synthesized via solution polycondensation of para-toluene sulfonate di-amines salts with activated di-acids. Typically dimethylsulfoxide or dimethylformamide are used as solvent. Typically as a base triethylamide is added, the reaction is carried out under an inert atmosphere at 60°C for 24-72 hours under constant stirring. Subsequently the obtained reaction mixture is purified via a water precipitation followed by an organic precipitation and filtration. Drying under reduced pressure yields the polyesteramide.

More specific the polyesteramide co-polymers of Formula (I) can be manufactured according to scheme 1. In scheme 1 specifically PEA-III-Bz of Formula (III) is manufactured.

As used herein, the term " articles " refer to microparticles. The articles are sized for injection which means they can be injected via a pharmaceutical syringe needle having a bore of about 18-30 Gauge. Stability of the articles is a critical factor for the injectability of the formulation. It is therefore extremely important that the articles such as for example microparticles remain stable and do not aggregate.

As used herein the term drug includes analgesics or disease-modifying antirheumatic drugs. The analgesics are preferably selected from the group of anti-inflammatory drugs, local anesthetic drugs or opioids. The anti-inflammatory drugs can be steroidal or non-steroidal anti-inflammatory drugs (NSAID).

Examples of steroidal anti-inflammatory agents include corticosteroids such as alclometasone dipropionate, amcinonide, amcinafel, amcinafide, beclamethasone, betamethasone, betamethasone dipropionate, betamethasone valerate, clobetasone propionate, chloroprednisone, clocortelone, Cortisol, cortisone, cortodoxone, difluorosone diacetate, descinolone, desonide, defluprednate, dihydroxycortisone, desoximetasone, dexamethasone, deflazacort, diflorasone, diflorasone diacetate, dichlorisone, esters of betamethasone, fluazacort, flucetonide, flucloronide, fludrotisone, fluorocortisone, flumethasone, flunisolide, fluocinonide, fluocinolone, fluocinolone acetonide, flucortolone, fluperolone, fluprednisolone, fluroandrenolone acetonide, fluocinolone acetonide, flurandrenolide, fluorametholone, fluticasone propionate, hydrocortisone, hydrocortisone butyrate, hydrocortisone valerate, hydrocortamate, loteprendol, medrysone, meprednisone, methylprednisone, methylprednisolone, mometasone furoate, paramethasone, paramethasone acetate, prednisone, prednisolone, prednidone, triamcinolone acetonide, triamcinolone hexacatonide, and triamcinolone, salts thereof, derivatives thereof, and mixtures thereof. As used herein, the term "derivative" refers to any substance which is sufficiently structurally similar to the material which it is identified as a derivative so as to have substantially similar functionality or activity, for example, therapeutic effectiveness, as the material when the substance is used in place of the material.

The most preferred corticosteroid in the formulation used for the treatment of arthritic disorders is triamcinolone acetonide or a commercially available chemical analogue or a pharmaceutically-acceptable salt thereof.

Examples of non-steroidal anti-inflammatory agents include ibuprofen, diclofenac, meloxicam, naproxen, etofenamaat, COX-2 inhibitors such as rofecoxib or celecoxib and derivatives thereof, and the like and mixtures thereof.

Most preferred non-steroidal anti-inflammatory agents in the formulation used for the treatment of arthritic disorders is a COX-2 inhibitor such as celecoxib or a commercially available chemical analogue or a pharmaceutically-acceptable salt thereof.

Examples of Opiods include codeine or morphine.

Examples of local anesthetics include Lidocaine or ropivacaine.

Examples of disease-modifying antirheumatic drugs are abatacept, adalimumab, azathioprine, chloroquine, D-penicillamine, etanercept, golimumab, infliximab, leflunomide, methotrexate, minocycline, rituximab or sulfasalazine, auranofin, cyclophosphamide or cyclosporine.

The formulation used for the treatment of arthritic disorders may comprise any suitable article that can be injected in a human or veterinary patient. In case that the article is a microparticle, the microparticle is sized such that it can be injected via a pharmaceutical syringe needle having a bore of about 18-30 Gauge.

The size of the microparticles may vary between 0.1-1000 micrometer. Typically, the average diameter of the microparticles given by the Fraunhofer theory in volume percent ranges from 100 nm to 1000 µm. The preferred average diameter may vary from 1-200 µm, more preferably from 5-80 µm. It is envisaged that particles with an average diameter of less than 1000 nm are nanoparticles. In particular, the particle diameter as used herein is the diameter as determinable by a Malven Mastersizer 2000. Particles can be defined and classified in various different ways depending on their specific structure, size, or composition, see e.g. Encyclopaedia of Controlled drug delivery Vol2 M-Z Index, Chapter: Microencapsulation Wiley Interscience, page 493-496. If particles are too small or non-analyzable by light scattering which may be the case with nanoparticles because of their optical properties, then scanning electron microscopy (SEM) or transmission electron microscopy (TEM) can be used to determine the particle diameter. The microparticles may comprise one or more analgesic. The analgesic(s) may be more or less homogeneously dispersed within the microparticles. The analgesic may also be located within the microparticle core or shell.

In addition to the biodegradable polyesteramides as represented by formula (I), the microparticles may further comprise one or more other polymers selected from the group of biocompatible polymers.

Examples of biocompatible polymers are poly(ortho esters), poly(anhydrides), poly(D,L-lactic acid), poly (L-lactic acid), poly(glycolic acid), copolymers of poly(lactic) and glycolic acid, poly(L-lactide), poly(D,L-lactide), poly(glycolide), poly(D,L-lactide-co-glycolide), poly(L-lactide-co-glycolide), poly(phospho esters), poly(trimethylene carbonate), poly(oxa-esters), poly(oxa-amides), poly(ethylene carbonate), poly(propylene carbonate), poly(phosphoesters), poly(phosphazenes), poly(tyrosine derived carbonates), poly(tyrosine derived arylates), poly(tyrosine derived iminocarbonates), copolymers of these polymers with poly(ethylene glycol) (PEG), or combinations thereof.

In principle the microparticles may be prepared in a manner known in the art, provided that the polymers used in the prior art are replaced by the biodegradable polyesteramides of Formula (I). In general particles can for example be prepared via aggregation with heat or pH adjustment, via co-acervation (phase separation), via spray drying or via solvent extraction. An overview of preparation methods has been disclosed in J. Control Release, 102:313-332, in 2005 by Freitas S et al. The microparticles used in the formulation of the present invention are preferably prepared via oil in water emulsion method. This method is disclosed in detail in Example 1.

If desired the microparticles may be loaded with one or more analgesics. Loading may be achieved by forming the microparticles in the presence of the analgesic or thereafter. To achieve microparticles with a high amount of analgesic, it is generally preferred to prepare the microparticles in the presence of the analgesic. In particular in the case that the analgesic is sensitive it is preferred to load the micro or nanoparticles after they have been formed. This can be achieved by contacting the microparticles with the analgesic and allowing the analgesic to diffuse into the microparticles and/or adhere/ adsorb to the surface thereof.

In accordance with the invention it is possible to provide microparticles with one or more analgesics with satisfactory encapsulation efficiency. (i.e. the amount of analgesic in the particles, divided by the amount of analgesic used). The loading efficiency is dependent on the analgesic used.

Several types of microparticle structures can be prepared, these include substantially homogenous structures. However in case that more than one analgesic has to be released or in case that one or more functionality is needed it is preferred that the microparticles are provided with a structure comprising an inner core and an outer shell. A core/shell structure enables more multiple mode of action for example in drug delivery of incompatible compounds or in imaging. The shell can be applied after formation of the core using a spray drier. The core and the shell may comprise the same or different polymers with different active agents. In this case it is possible to release the bioactive agents at different rates. It is also possible that the bioactive agent is only present in the core and that the shell is composed of a polymer.

The microparticles can also be present in a spray-able form as a suspension in a free form or in an in-situ forming gel formulation.

The formulation is used for the treatment of arthritic disorders, preferably arthritis more preferably osteoarthritis, most preferably osteoarthritis of the knee. The formulation used for the treatment of arthritic disorders is preferably administered in 1 or 2 injections per year. The scope of the invention is defined by the claims. Any references in the description to methods of treatment refer to the compounds, pharmaceutical compositions and medicaments of the present invention for use in a method for treatment of the human (or animal) body by therapy (or for diagnosis). The present invention also relates to method of treating pain or inflammation in a human or veterinary patient comprising administering to said patient a therapeutically effective amount of the formulation.

The present invention moreover relates to a method of slowing, arresting or reversing progressive structural tissue damage associated with chronic inflammatory disease in a human or veterinary patient comprising administering to said patient a therapeutically effective amount of the formulation. The formulation is preferably administered in 1 or 2 injections per year.

The present invention will now be described in detail with reference to the following non limiting Figures and examples which are by way of illustration only.

### FIGURES

FIG. 1: SEM photograph of microparticles
FIG. 2: Size distribution of a plurality of microparticles
FIG 3: Shows the biological effect in inflammation reduction (cell-assay based in vitro experiment. PGE-2 levels from human chondrocytes in presence of PEA-III-Bz particles.
FIG 4: Cumulative fluorescein release from PEA-III-Bz films. Effect of the inflammatory cells lysate: stage 1 diffusion driven release, stage 2 in presence of (solution of broken inflammatory cells provides lysate)lysate, stage 3 diffusion driven release after partial degradation by lysates.
FIG 5: Fluorescein release rate. Effect of the inflammatory cells lysate.
FIG 6: Size distribution by light scattering of PEA-I-Bz microparticles after being immersed in water for 10 minutes. The second pic of the size distribution is a sign of agglomeration of particles
FIG 7: Size distribution by light scattering of PEA-I-Bz microparticles after being immersed in water for 400 minutes. We can observed and increased of large particles as well as a widening of the size distribution compared to the measurement performed at 10 minutes post immersion.
FIG 8: Size distribution by light scattering of PEA-III-Bz microparticles after being immersed in water for 10 minutes
FIG 9: Size distribution by light scattering of PEA-III-Bz microparticles after being immersed in water for 400 minutes

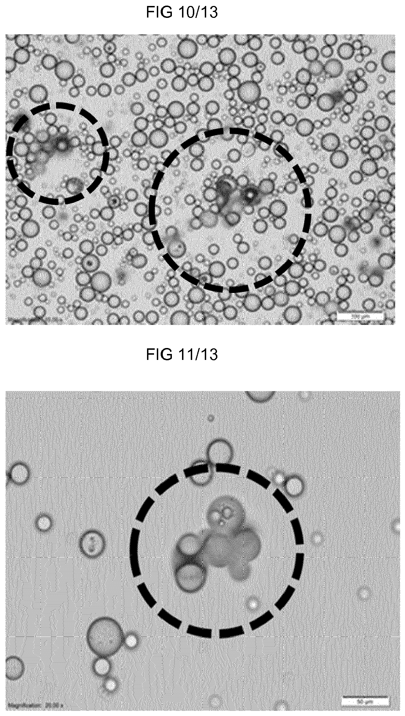

FIG 10: (light) microscopic representation of microparticles of PEA-I-Bz after being immersed in water for 10 minutes. Agglomerates are circled.
FIG 11: (light) microscopic representation of microparticles of PEA-I-Bz after being immersed in water for 400 minutes. Agglomerates are circled.

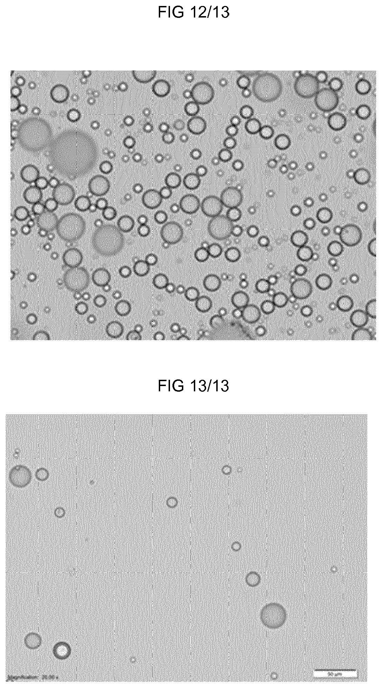

FIG 12: (light) microscopic representation of microparticles of PEA-III-Bz after being immersed in water for 10 minutes. No agglomerates of particles are observed.
FIG 13: (light) microscopic representation of microparticles of PEA-III-Bz after being immersed in water for 400 minutes. No agglomerates of particles are observed.

### MATERIALS

- PGE-2 concentration is compared to levels produced in normal condition (Control - experiment).
- Presence of TnF-α increases the production of PGE-2 from chondrocytes (experiment Control+) to 300%
- Presence of empty particles does not influence on the production of PGE-2 from chondrocyte compare to Control+ experiment.
- 0.1 µM of TAA stops the production of PGE-2 by chondrocytes. This illustrates that Triamcinolone acetonide reduces inflammation.
- Chondrocytes incubated with TAA-loaded Microparticles do not produce PGE-2. This conditions shows that microparticles release an active agent that limits the inflammation of human chondrocyte, this, even when stimulated by TnF-α.
- PEA-III-Bz polymers are used in the following examples. A more extended description of PEA-III-Bz is poly-8-[(L-Leu-DAS)_{0.45}(L-Leu-6)_{0.3}-[L-Lys(Bz)]_{0.25}. Structure is given in Formula III. The fractions indicate overall fractions of the monomers in the synthesis given in scheme 1.
- PEA-I-Bz polymers are used in microparticles for comparison with PEA-III-Bz microparticles. Structure of PEA-I-Bz polyesteramides is given in below Formula (IV). 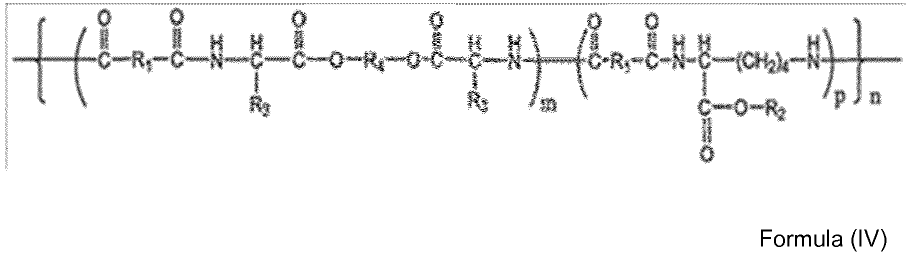 Wherein m varies from 0.1 to 0.9; p varies from 0.9 to 0.1; n varies from 50 to 150;
   - each R1 is independently (C1 -C₂₀)aikyiene; each R₂ is independently hydrogen, or (C₆-C₁₀)aryl(C1-C₆)alkyl such as benzyl.
   - each R₃ is independently hydrogen, (C1-C₆) alkyl, (C₂-C₆)alkenyl, (C₂-C₆)alkynyl, or (C₆-C₁₀)aryl(C₁-C₆)alkyl; and each R4 is independently (C₂-C₂₀)alkylene.

PEA-I is a copolymer comprising alpha -amino acids, diols and an aliphatic dicarboxylic acids, which is copolymerized with aliphatic dicarboxylic acid and lysine.

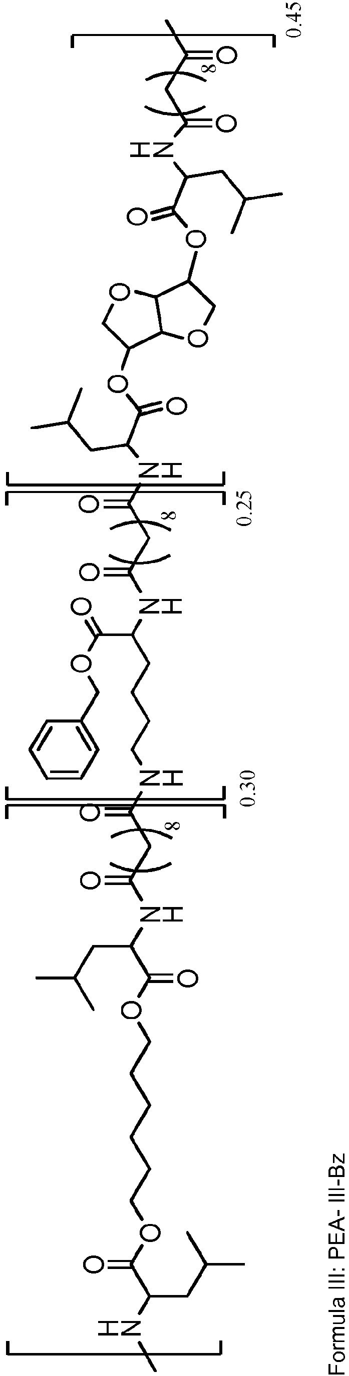

### SYNTHESIS of PEA-III-Bz

Trietylamine (30.9 mL, 0.222 mole, 2.2eq) and N,N-dimethylformamide (53.07 mL, 0.689mole) were added to a mixture of Di-OSu-sebacinate (39.940 g, 0.1008 mole, 1.0eq), L-leucine(6)-2TosOH (20.823 g, 0.0302 mole, 0.30eq), L-leucine-(DAS)-2TosOH (32.503 g, 0.0453 mole, 0.45eq) and L-lysine(Bz)-2TosOH (14.628 g, 0.0252 mole, 0.25eq) in a nitrogen flushed 500mL round bottomed flask equipped with a overhead stirrer at room temperature. The subsequent mixture was heated to 60°C to allow the reaction to proceed and monitored by GPC analysis in THF. After 36 hours a stable molecular weight was obtained, subsequently a portion of L-leucine(6)-2TosOH (4.338 g, 0.0063 mole) along with triethylamine (1.76 mL, 0.0126mole) and N,N-dimethylformamide (4.54 mL, 0.0590mole) was added to terminate the polymerization reaction. The mixture was heated additionally for 24 hours after which the viscous solution was further diluted with N,N-dimethylformamide (407.85g, 5.301mole) and allowed to cool to room temperature. At room temperature acetic anhydride (1.89 mL, 0.0199mole) was added to acylate the amino functional end groups of the polymer. The mixture was stirred at room temperature for 24 hours. In scheme 1 the general reaction is shown.

The obtained crude polymer mixture was precipitated in water in a 10:1 ratio (water: reaction mixture). The polymer was collected and dissolved in ethanol (500 mL, 8.57mole) and the procedure was repeated a second time. The polymer was again dissolved in ethanol (500 mL, 8.57mole) and precipitated in ethylacetate (5000 mL, 50.91mole) by drop wise addition to a stirring solution. The precipitated polymer was washed with two portions ethylacetate (100mL, 1.00mole), dried and dissolved in ethanol (500mL, 8.57mole) and filtered over a 0.2µm PTFE membrane filter. The filtered polymer solution was dried under reduced pressure at 65°C.

Yield 75%, Mn =50 kDa (Gel Permeation Chromatography (GPC) in THF relative to polystyrene standards. Glass transition temperatures were determined by Differential Scanning Calorimetry (DSC). Measurements were taken from second heating, with a heating rate of 10°C. /min., Tg= 48°C.

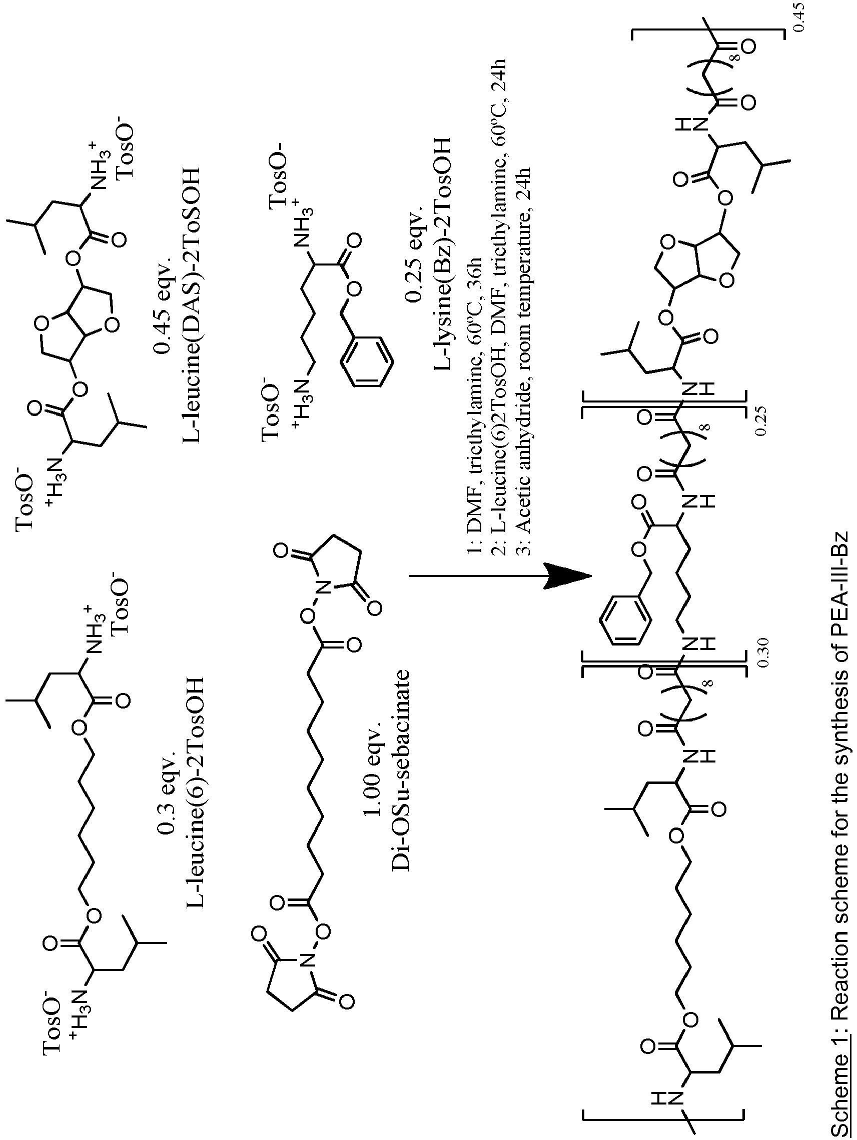

### PRINCIPLE ON DEGRADATION CONTROLLED RELEASE.

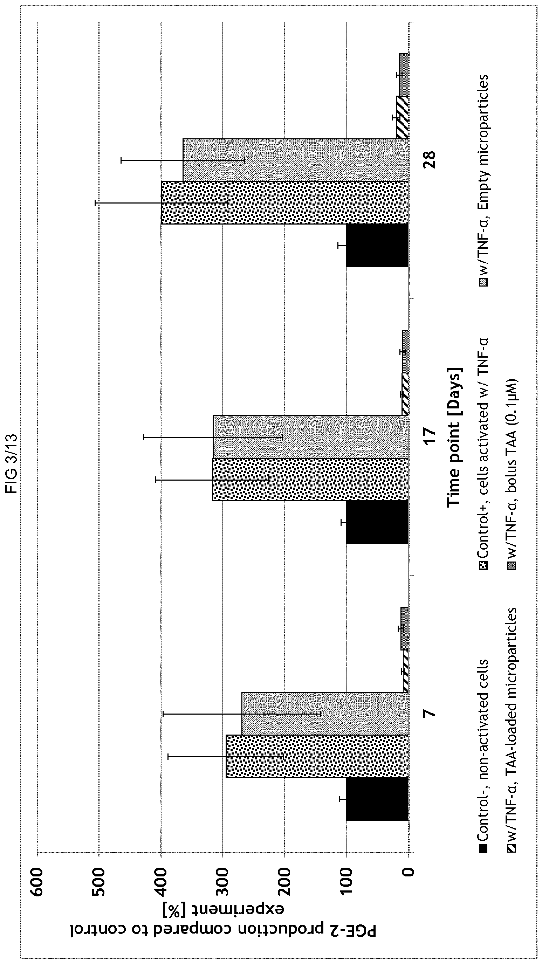

Drug loaded microspheres with TAA as a drug are used in the study. As reference diffusion driven release has been performed in cell culture medium.. The expected release curve is anticipated to closely resemble hypothetical figure 3A.

Second and third series of the same particle batch (also in triplicate) will be started at the same time. The first phase of the release will be performed similar to the control experiment (stage 1 in figure 3 B). At stage 2 the cell culture medium will be replaced with cell culture medium containing HL-60 neutrophil like cell lysates. To series two pure lysate will be added, to series 3 10X diluted lysate will be added. The enzymes present in the lysate will cause surface degradation of PEA microparticles and as a result an increase in the release of API is anticipated..

### EXAMPLES

### Example 1

### Fabrication of TAA-loaded PEA-III-Bz microparticles.

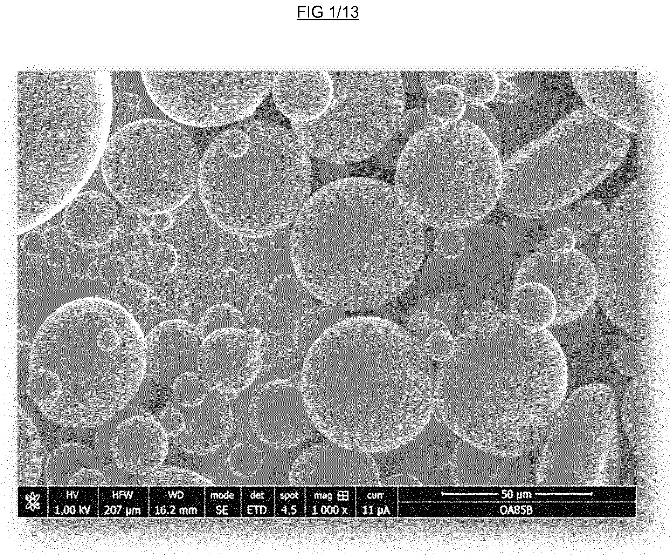

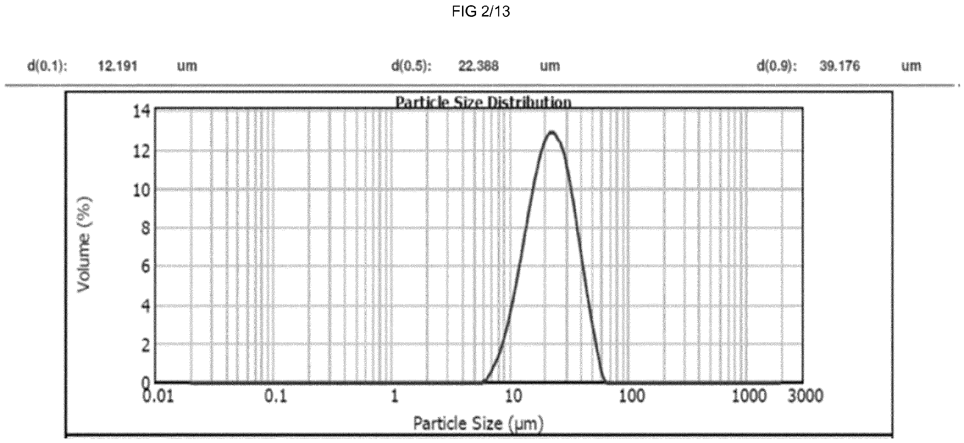

300mg of PEA-III-Bz was dissolved in Dichloromethane. 75mg of TAA was added to the solution and homogenized by ultrasound. The suspension was added to 20ml of cold water containing 1wt% of poly(vinyl alchohol) under high shear, using a ultra-Turrax®. After a stable suspension was obtained the particles were let hardened in 100ml of water containing 1wt% of poly(vinyl alchohol) for 12 hours. Excess of water and surfactant was removed by rinsing and centrifugation. Finally, particles were frozen and dried under vacuum. A picture of the microparticles is given in figure 1. The size distribution of the microparticles is given in figure 2.

### Example 2

### Culture of chondrocyte in the presence of TAA-loaded PEA-III- Bz microparticles.

OA chondrocytes from three human donors were harvested and cultivated from total knee replacement. The cells were incubated with 5µg of microparticles produced in example 1 in a transwells. Every three days the cells and medium were collected and the transwells were transferred to wells with freshly plated OA chondrocytes from the same donor. Quantities of PGE-2 (prostaglandin-E 2) produced by the cells during the time of incubation were determined by ELISA.
The total duration of the experiment was of 28 days.
Cells were cultured in the following conditions:
1. Control experiment; without particles
2. Positive control, chondrocytes stimulated by TNF-alpha, without particles
3. Chondrocytes stimulated by TNF-alpha, incubated with 5µg of empty PEA-III-Bz particles
4. Chondrocytes stimulated by TNF-alpha, incubated with 5µg of PEA-III- Bz particles loaded with 20wt% of Triamcinolone acetonide
5. Chondrocytes stimulated by TNF-alpha, incubated with a constant concentration of Triamcinolone acetonide of 0.1µM
Results are represented in figure 3.

### Example 3

### Cumulative release of fluorescein from PEA-III-Bz films

A proof of principle study has been executed with fluorescein as a dye that is slowly released from a film.

During acute inflammation, polymorphonuclear neutrophil cells (PMNs) are the first type of cells to migrate to an inflammatory site, where they will produce several pro-inflammatory mediators including chemokines that attract other PMNs and other cell types like monocytes-macrophages and lymphocytes, corresponding to chronic inflammation. Neutrophils and macrophages are two cells types known to interact with biomaterials, which could lead to possible degradation of a biomaterial. Modelling the interaction of neutrophil like cells with a biomaterial can be done with differentiated HL60 neutrophil like cells. Neutrophils are the first-responders of inflammatory cells to migrate toward the site of inflammation and are known to have a high level of interaction with foreign bodies such as biomaterials.

The effect of acute inflammation on the release of fluorescein was assessed on 10wt% fluorescein loaded PEA-III-Bz films. A comparison release series was performed in PBS buffer with 0.05wt% sodium azide, sodium azide was added as a biocide. The effect of inflammation on the release of the dye (fluorescein) was demonstrated by the addition of cell lysates from differentiated HL60 neutrophil like cells.

### Samples preparation

101.3mg fluorescein and 999.3mg PEA-III-Bz were dissolved in 19ml ethanol. The solution was left overnight to dissolve under gentle agitation on an orbital shaker.
8ml of the polymer fluorescein solution was pipetted in a Teflon mold with a diameter of 5cm placed in a desiccator. Under a gentle nitrogen flow the solvent was allowed to evaporate in 18 hours. The nitrogen flow dried films were removed and dried further under vacuum at 70°C for 48 hours. A sample of the film was analyzed for residual ethanol by 1H-NMR analysis in CDCl3, no ethanol peaks were detected. 6mm round disks were punched out of the dried film and were used for the release experiment.

### Release experiment

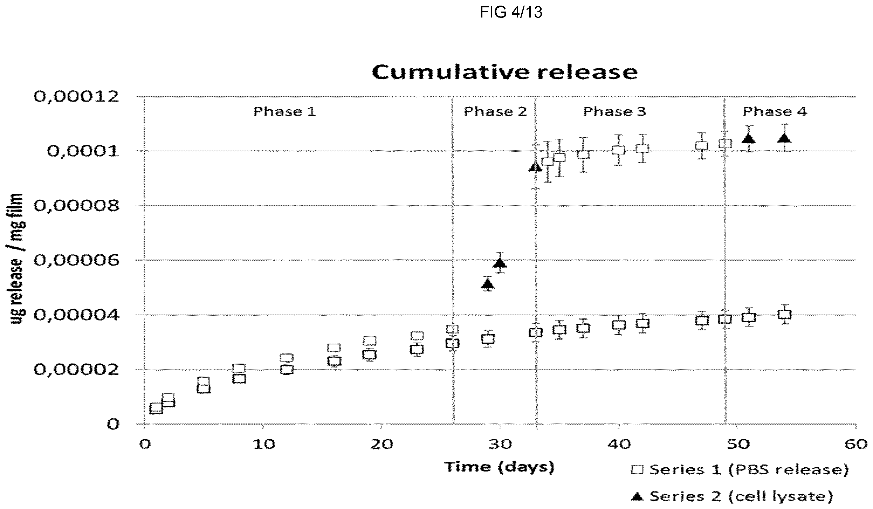

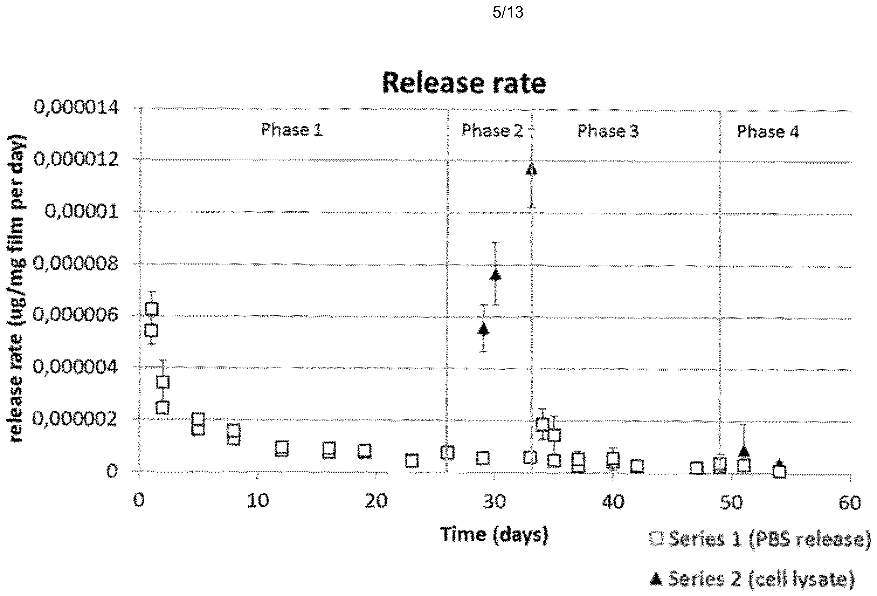

Two release series were started both in triplicate. At each time point the solutions were refreshed. Results are shown in figures 4 and 5.

### Principle of inflammation induced release

Series 1 released in PBS buffer for the entire period (diffusion driven release). The release of fluorescein in series 2 is divided in four distinct phases.

Phase 1 release was performed in PBS buffer similar to series 1. In this phase both release curves closely resemble each other.

Phase 2, after 26 days of release HL60 neutrophil like cell lysate was added to the samples of series 2. The addition of the lysate results in an increased release rate of fluorescein.

Phase 3, after 33 days the action of the lysate was eliminated by a pre-treatment with 4-(2-Aminoethyl)benzenesulfonyl fluoride hydrochloride. The release rate in this phase closely resembled the release in PBS buffer. In phase 4 again fresh lysate was added and the release rate increased before the entirely fluorescein load to be released. See fig. 4 for the cumulative release of fluorescein and fig. 5 for the release rate.

The experiment clearly shows the surprising effect of the inflammation cell lysate on the release rate of from the polymer matrix. The results of the model system suggest that the release of an API from PEA III Ac Bz can be interactively tuned by the level of inflammation for a patient benefit.

Strong inflammation→Increased release rate→slowing down the inflammation→lower release rate.

### Example 4

### Fabrication of PEA-III-Bz microparticles as described in Example 1 Fabrication PEA-I microparticles.

300mg of PEA-I-Bz was dissolved in Dichloromethane. The suspension was added to 20ml of cold water containing 1wt% of poly(vinyl alchohol) under high shear, using a ultra-Turrax®. After a stable suspension was obtained the particles were let hardened in 100ml of water containing 1wt% of poly(vinyl alchohol) for 12 hours. Excess of water and surfactant was removed by rinsing and centrifugation. Finally, particles were frozen and dried under vacuum.

### Stability of PEA microparticles in water at room temperature; agglomeration of PEA-I-Bz vs PEA-III-Bz

30mg of PEA-III-Bz and PEA-I-Bz microspheres were suspended with 0.5ml water and put on a shaker at 20°C at 120rpm. Size and agglomeration of microparticles were monitored by size distribution measurement (using light scattering technic) and visual evaluation (light microscopic technic) for both type of particles after 10 and 400 minutes of immersion in water.

It could be seen that while PEA-III-Bz did not agglomerated during the course of the experiment, PEA-I-Bz particles were showing agglomeration after only 10 minutes of immersion in water. Agglomerated particles reduces the syringe-ability of those particles through a narrow needle.

### Particle size measurement by light scattering after 10 and 400 minutes;

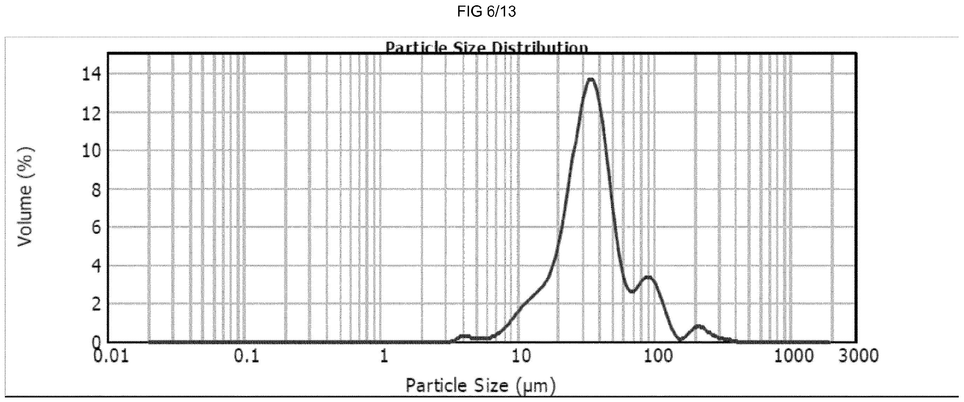

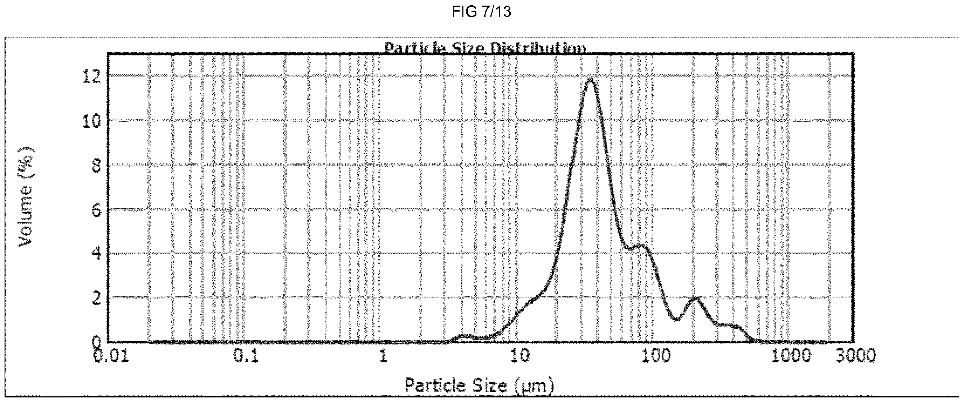

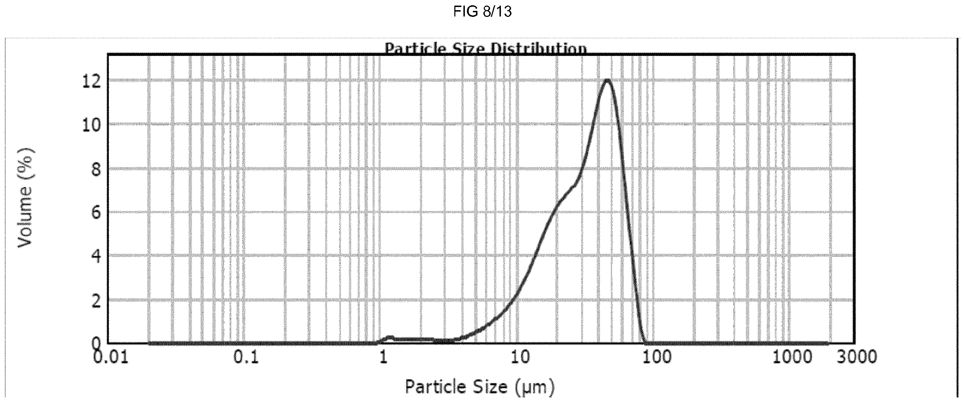

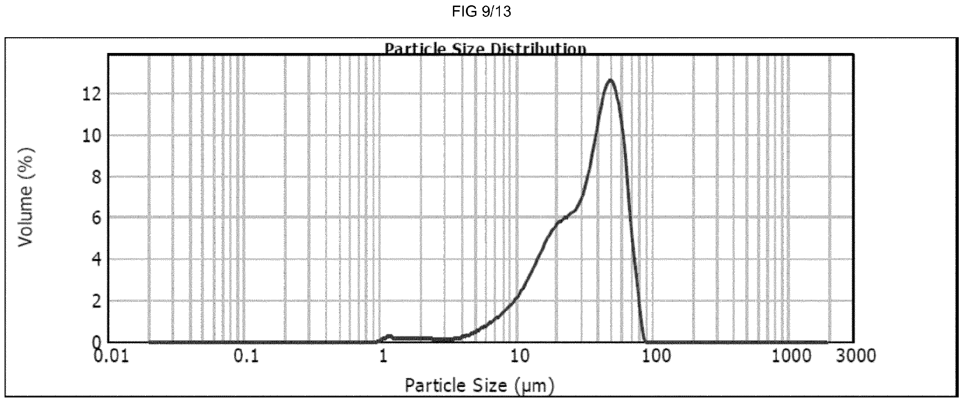

The size distribution is defined by D10, D50, D90 and SPAN; where D10 corresponds to the value of the particle diameter at 10% in cumulative distribution, where D50 corresponds to the value of the particle diameter at 50% in cumulative distribution and where D90 corresponds to the value of the particle diameter at 90% in cumulative distribution as can be seen in the figures 6-9.

The SPAN is calculated as SPAN=(D90-D10)/D50.

### PEA-I-Bz

| Duration of immersion | D(10) | D(50) | D(90) | SPAN |
|---|---|---|---|---|
| 10 min | 16.177 | 34.513 | 85.802 | 2.017 |
| 400 min | 18.653 | 39.393 | 127.754 | 2.77 |

Agglomeration is clearly visible by an increase of all values in time.

### PEA-III-Bz

| Duration of immersion | D(10) | D(50) | D(90) | SPAN |
|---|---|---|---|---|
| 10min | 12.001 | 33.667 | 59.064 | 1.398 |
| 400min | 12.158 | 36.432 | 62.236 | 1.375 |

With respect to experimental accuracy, PEA-III-Bz microparticles do not show evidence of agglomeration after 400 minutes of immersion in water.

## Claims

1. A formulation injectable in a human or veterinary patient for use in the treatment of an arthritic disorder comprising microparticles sized for injection, wherein the microparticles comprise an analgesic or a disease-modifying antirheumatic drug, and one or more biocompatible polymers comprising a biodegradable polyesteramide co-polymer comprising structural formula (I): 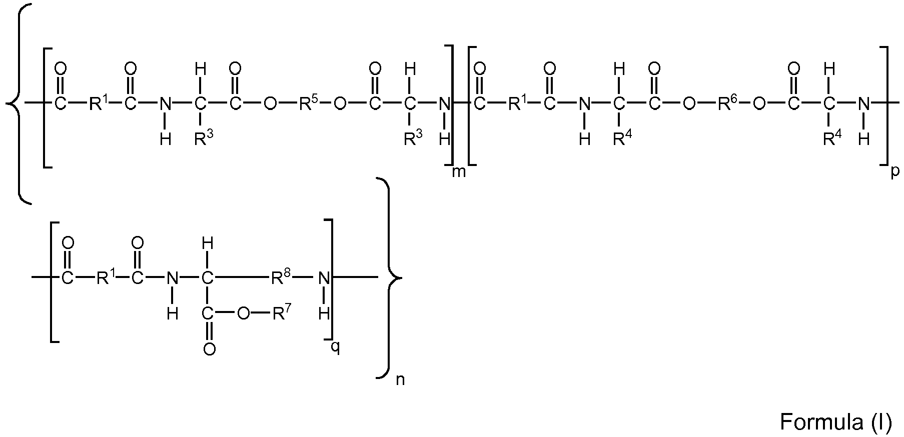 wherein
- m varies from 0.01- 0.99; p varies from 0.99-0.01; and q varies from 0.99-0.01;
- n varies from 5-100;
- R₁ is independently selected from the group consisting of (C₂-C₂₀)alkylene, (C₂-C₂₀)alkenylene and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl (C₁-C₆)alkyl, -(CH₂)SH,-(CH₂)₂S(CH₃), -CH₂OH, -CH(OH)CH₃, -(CH₂)₄NH₃+, -CH₂COOH,-(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, phenyl-CH₂-,-CH=CH-CH₃, HO-p-phenyl-CH₂-, (CH₃)₂-CH-, phenyl-NH-, NH₂-(CH₂)₃-CH₂- or NH₂-CH=N-CH=C-CH₂-;
- R₅ is selected from the group consisting of (C₂-C₂₀) alkylene, (C₂-C₂₀) alkenylene;
- R₆ is selected from bicyclic-fragments of 1,4:3,6-dianhydrohexitols of structural formula (II); 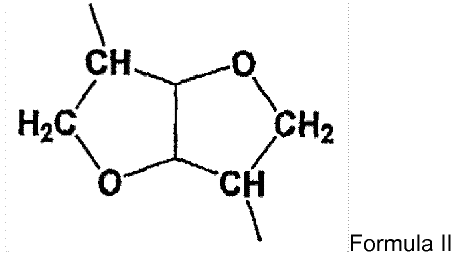
- R₇ is hydrogen, (C₆-C₁₀) aryl, (C₁-C₆) alkyl or a protecting group such as benzyl; and
- R₈ is independently (C₁-C₂₀) alkylene or (C₂-C₂₀)alkenyl.

2. The formulation according to claim 1, wherein the polyesteramide co-polymer of Formula (I) comprises m+p+q=1, q=0.25, p=0.45 whereby R₁ is -(CH₂)₈-; R₃ and R₄ in the backbone units m and p is (CH₃)₂-CH-CH₂-, R₅ is -(CH₂)₆-, R₆ is a bicyclic-fragment of 1,4:3,6-dianhydrohexitols of structural formula (II); R₇ is a benzyl group and R₈ is -(CH₂)₄-.

3. The formulation according to claim 1, wherein R₇ is benzyl.

4. The formulation according to any one of claims 1-3, wherein the microparticles comprise an analgesic and the analgesic is selected from anti-inflammatory drugs, local anesthetic drugs and opioids.

5. The formulation according to any one of claims 1-4, wherein the microparticles comprise an analgesic and the analgesic is a NSAID COX-2 inhibitor.

6. The formulation according to any one of claims 1-4, wherein the microparticles comprise an analgesic and the analgesic is a corticosteroid.

7. The formulation according to claim 6, wherein the corticosteroid is triamcinolone acetonide.

8. The formulation according to any one of claims 1-3, wherein the microparticles comprise a disease-modifying antirheumatic drug.

9. The injectable formulation according to any one of claims 1-8, wherein the size of the microparticles are from 0.1 to 1000 micrometer.

10. The formulation according to any one of the claims 1-9 for use in the treatment of arthritis.

11. The formulation according to any one of claims 1-10 for use in the treatment of osteoarthritis.

12. The formulation according to any one of claims 1-11 for use in the treatment of osteoarthritis of the knee, hip, spine or shoulder.

13. The formulation according to any one of claims 1-12 for use in the treatment of arthritic disorders, wherein the formulation is administered in 1 or 2 injections per year.

## Patentansprüche

1. Formulierung, injizierbar in einen menschlichen oder veterinären Patienten, zur Verwendung bei der Behandlung einer arthritischen Störung, umfassend Mikropartikel, die für die Injektion bemessen sind, wobei die Mikropartikel ein analgetisches oder krankheitsmodifizierendes antirheumatisches Arzneimittel und ein oder mehrere biokompatible Polymere umfassen, die ein biologisch abbaubares Polyesteramidcopolymer umfassen, umfassend die Strukturformel (I): 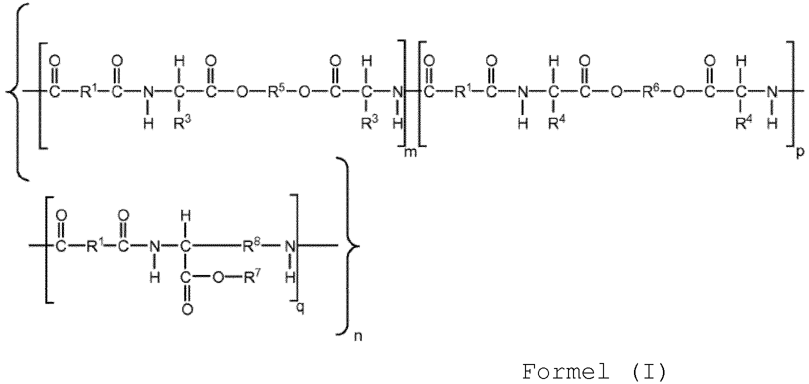 worin
- m von 0,01-0,99 variiert; p von 0,99-0,01 variiert; und q von 0,99-0,01 variiert;
- n von 5-100 variiert;
- R₁ unabhängig aus der Gruppe bestehend aus (C₂-C₂₀) Alkylen, (C₂-C₂₀) Alkenylen und Kombinationen davon ausgewählt ist;
- R₃ und R₄, jeweils in einer einzelnen Grundgerüsteinheit m oder p, unabhängig ausgewählt sind aus der Gruppe bestehend aus Wasserstoff, (C₁-C₆)Alkyl, (C₂-C₆)Alkenyl, (C2-C6)Alkinyl, (C₆-C₁₀)Aryl(C₁-C₆)alkyl,-(CH₂)SH, -(CH₂)₂S(CH₃), -CH₂OH, -CH(OH)CH₃, -(CH₂)₄NH₃+,-CH₂COOH, -(CH₂)COOH, -CH₂-CO-NH₂, -CH₂CH₂-CO-NH₂,-CH₂CH₂COOH, CH₃-CH₂-CH(CH₃)-, (CH₃)₂-CH-CH₂-, H₂N-(CH₂)₄-, Phenyl-CH₂-, -CH=CH-CH₃, HO-p-Phenyl-CH₂-, (CH₃)₂-CH-, Phenyl-NH-, NH₂-(CH₂)₃-CH₂- oder NH₂-CH=N-CH=C-CH₂-;
- R₅ ausgewählt ist aus der Gruppe bestehend aus (C₂-C₂₀)Alkylen, (C₂-C₂₀)Alkenylen;
- R₆ ausgewählt ist aus bizyklischen Fragmenten von 1,4:3,6-Dianhydrohexitolen von Strukturformel (II); 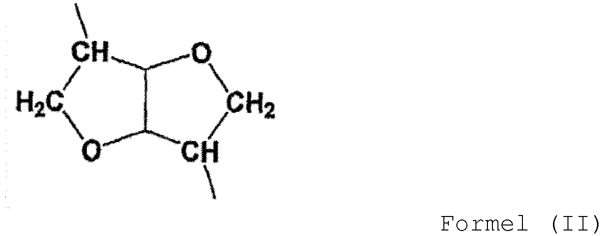
- R₇ Wasserstoff, (C₆-C₁₀)Aryl, (C₁-C₆)Alkyl oder eine Schutzgruppe, wie Benzyl, ist; und
- R₈ unabhängig (C₁-C₂₀)Alkylen oder (C₂-C₂₀)Alkenyl ist.

2. Formulierung nach Anspruch 1, wobei das Polyesteramid-Copolymer der Formel (I) m+p+q=1, q=0,25, p=0,45 umfasst, wobei R₁-(CH₂)₈- ist; R₃ und R₄ in den Grundgerüsteinheiten m und p für (CH₃)₂-CH-CH₂- steht, R₅-(CH₂)₆-ist, R₆ ein bicyclisches Fragment von 1,4:3,6-Dianhydrohexitolen der Strukturformel (II) ist; R₇ eine Benzylgruppe ist und R₈-(CH₂)₄- ist.

3. Formulierung nach Anspruch 1, wobei R₇ Benzyl ist.

4. Formulierung nach einem der Ansprüche 1-3, wobei die Mikropartikel ein Analgetikum umfassen und das Analgetikum ausgewählt ist aus entzündungshemmenden Arzneimitteln, Lokalanästhetika und Opioiden.

5. Formulierung nach einem der Ansprüche 1-4, wobei die Mikropartikel ein Analgetikum umfassen und das Analgetikum ein NSAID-COX-2-Inhibitor ist.

6. Formulierung nach einem der Ansprüche 1-4, wobei die Mikropartikel ein Analgetikum umfassen und das Analgetikum ein Corticosteroid ist.

7. Formulierung nach Anspruch 6, wobei das Corticosteroid Triamcinolonacetonid ist.

8. Formulierung nach einem der Ansprüche 1-3, wobei die Mikropartikel ein krankheitsmodifizierendes antirheumatisches Arzneimittel umfassen.

9. Injizierbare Formulierung nach einem der Ansprüche 1 bis 8, wobei die Größe der Mikropartikel 0,1 bis 1000 Mikrometer beträgt.

10. Formulierung nach einem der Ansprüche 1-9 zur Verwendung bei der Behandlung von Arthritis.

11. Formulierung nach einem der Ansprüche 1-10 zur Verwendung bei der Behandlung von Osteoarthritis.

12. Formulierung nach einem der Ansprüche 1-11 zur Verwendung bei der Behandlung von Osteoarthritis des Knies, der Hüfte, der Wirbelsäule oder der Schulter.

13. Formulierung nach einem der Ansprüche 1-12 zur Verwendung bei der Behandlung von arthritischen Störungen, wobei die Formulierung in 1 oder 2 Injektionen pro Jahr verabreicht wird.

## Revendications

1. Formule injectable à un patient humain ou vétérinaire pour utilisation dans le traitement d'un trouble arthritique comprenant des microparticules dimensionnées pour injection, où les microparticules comprennent un analgésique ou un médicament antirhumatique modifiant la maladie, ainsi qu'un ou plusieurs polymères biocompatibles comprenant un copolymère de polyesteramide biodégradable répondant à la formule structurelle (I) : 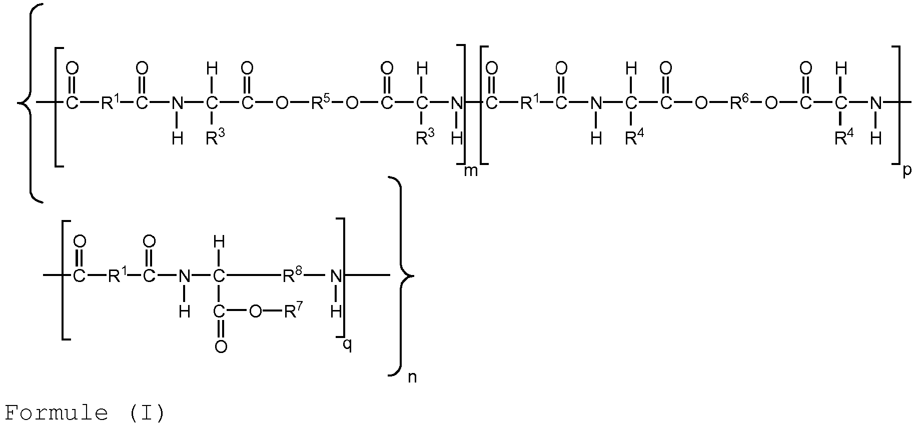 où
- m varie entre 0,01 et 0,99 ; p varie entre 0,99 et 0,01 ; et q varie entre 0,99 et 0,01 ;
- n varie entre 5 et 100 ;
- R₁ est indépendamment choisi dans le groupe constitué par les groupements alkylène en C₂-C₂₀, alcénylène en C₂-C₂₀ et leurs combinaisons ;
- chacun des radicaux R₃ et R₄, respectivement dans un motif de squelette unitaire m ou p, est indépendamment choisi dans le groupe constitué par l'atome d'hydrogène et les groupements alkyle en C₁-C₆, alcényle en C₂-C₆, alcynyle en C₂-C₆, (aryle en C₆-C₁₀)-(alkyle en C₁-C₆), -(CH₂)SH, -(CH₂)₂S(CH₃), -CH₂OH,-CH(OH)CH₃, -(CH₂)₄NH₃+, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, phényl-CH₂-, -CH=CH-CH₃, HO-p-phényl-CH₂-, (CH₃)₂-CH-, phényl-NH-, NH₂-(CH₂)₃-CH₂- ou NH₂-CH=N-CH=C-CH₂- ;
- R₅ est choisi dans le groupe constitué par les groupements alkylène en (C₂-C₂₀) et alcénylène en (C₂-C₂₀) ;
- R₆ est choisi parmi les fragments bicycliques de 1,4 : 3,6-dianhydrohexitols de formule structurelle (II) ; 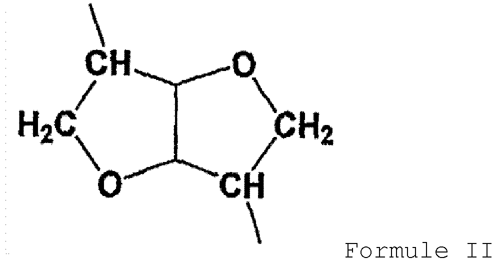
- R₇ représente un atome d'hydrogène ou un groupement aryle en C₆-C₁₀, alkyle en C₁-C₆ ou un groupement protecteur tel que benzyle ; et
- R₈ représente indépendamment un groupement alkylène en C₁-C₂₀ ou alcényle en C₂-C₂₀.

2. Formule selon la revendication 1, où le copolymère de polyesteramide de Formule (I) comprend m + p + q = 1, q = 0,25, p = 0,45 dont il résulte que R₁ représente un groupement -(CH₂)₈- ; chacun des radicaux R₃ dans les motifs de squelette m et p représente un groupement (CH₃)₂-CH-CH₂-, R₅ représente un groupement-(CH₂)₆-, R₆ représente un fragment bicyclique de 1,4 : 3,6-dianhydrohexitols de formule structurelle (II) ; R₇ représente un groupement benzylique et R₈ représente-(CH₂)₄-.

3. Formule selon la revendication 1, où R₇ représente un groupement benzyle.

4. Formule selon l'une quelconque des revendications 1 à 3, où les microparticules comprennent un analgésique et l'analgésique est choisi parmi les médicaments anti-inflammatoires, les anesthésiques locaux et les opioïdes.

5. Formule selon l'une quelconque des revendications 1 à 4, où les microparticules comprennent un analgésique et l'analgésique est un AINS inhibiteur de COX-2.

6. Formule selon l'une quelconque des revendications 1 à 4, où les microparticules comprennent un analgésique et l'analgésique est un corticostéroïde.

7. Formule selon la revendication 6, où le corticostéroïde est le triamcinolone acétonide.

8. Formule selon l'une quelconque des revendications 1 à 3, où les microparticules comprennent un médicament antirhumatique modifiant la maladie.

9. Formule pour injection selon l'une quelconque des revendications 1 à 8, où la taille des microparticules est comprise entre 0,1 et 1000 micromètres.

10. Formule selon l'une quelconque des revendications 1 à 9, pour utilisation dans le traitement de l'arthrite.

11. Formule selon l'une quelconque des revendications 1 à 10, pour utilisation dans le traitement de l'arthrose.

12. Formule selon l'une quelconque des revendications 1 à 11 pour utilisation dans le traitement de l'arthrose du genou, de la hanche, de la colonne vertébrale ou de l'épaule.

13. Formule selon l'une quelconque des revendications 1 à 12 pour utilisation dans le traitement des troubles arthritiques, où la formule est administrée en 1 ou 2 injections par an.
